# EUROPEAN PATENT SPECIFICATION

(11) **EP 3 240 509 B1**
(45) Date of publication and mention of the grant of the patent: **09.09.2020**
(21) Application number: 15823100.1
(22) Date of filing: 18.12.2015
(51) Int. Cl.: A61F 5/01

(54) **WATERPROOF, POLYMERIC BRACE**
WASSERDICHTE POLYMERSTÜTZE
ATTELLE POLYMÈRE IMPERMÉABLE À L'EAU

(30) Priority: 30.12.2014 US 201462098081 P
(43) Date of publication of application: 08.11.2017
(73) Proprietor: 3M Innovative Properties Company, St. Paul, MN 55133-3427 (US)
(72) Inventor: HINDS, Sherry A., Saint Paul, Minnesota 55133-3427 (US); WEAVER, Edward L., II, Saint Paul, Minnesota 55133-3427 (US)
(74) Representative: Vossius & Partner Patentanwälte Rechtsanwälte mbB
(86) International application number: PCT/US2015/066779
(87) International publication number: WO 2016/109258

(56) References cited:
- US-A- 5 306 229
- US-A- 6 093 161
- US-A1- 2005 010 155
- US-A1- 2010 036 300
- US-B1- 6 517 501

## Description

### SUMMARY

Common recreational orthopedic supports are constructed of natural or synthetic fabrics or other textile materials, either alone or laminated to more rigid materials. These textile materials are permeable to the elements, including water, dirt, salt (from perspiration) or bacteria. Moreover, such supports are often impractical for certain high impact or moisture heavy occupations. Other orthopedic supports, such as those described in US Patent No. 6,093,161 (Vlaeyen et al.), include thermoplastic materials that must be heated and cooled to conform to the wearer's anatomy. Such supports are especially reliant on sophisticated intermediaries to form the support or especially compliant users.

The present invention provides an orthopedic support according to claim 1. Preferred embodiments are defined in the dependent claims.

The present disclosure further provides preformed orthopedic devices that lack any textile material, yet provide the desired wearer comfort and anatomical support. Advantageously, the orthopedic supports can be delivered to wearer preformed to contour the curvature of the wearer's anatomy. The materials utilized to manufacture the orthopedic supports of the present disclosure can provide resistance to impact, compression set, and tearing while offering flexibility/stretch in unsupported directions. In addition, because the material by which it is fabricated is or is made breathable, the orthopedic support is more comfortable to wear as it allows perspiration and evaporation. Skin irritation and skin maceration can advantageously be diminished. In a further advantage, the orthopedic support is at least washable, if not water proof.

In one aspect, the present disclosure provides an orthopedic support including a main brace body comprising first and second opposing sides, the brace body consisting essentially of a unitary sheet of elastomeric material, the unitary sheet formed to at least partially encircle and generally conform to a joint of a wearer, wherein the layer includes an inner surface and an exterior surface, and wherein the body includes a plurality of engineered apertures extending through the sheet from the exterior surface to the inner surface. The brace may further include a structural support removably received in the exterior surface of the main body, the structural support having a stiffness greater than the stiffness of the unitary sheet.

In another aspect, the present disclosure provides a wrist brace including a unitary sheet of flexible, formed foam having an inner surface, an outer surface, a distal edge, proximal edge and opposite lateral edges, the sheet being preset in a configuration designed to encircle a portion of a wearer's wrist and forearm, wherein the unitary sheet has an Asker C hardness of at least 30 and no greater than 55, and wherein the neither the inner surface nor the exterior surface is not coupled to a textile material.

In another aspect, the present disclosure provides an orthopedic support including a main brace body comprising first and second opposing sides, the brace body consisting essentially of a unitary sheet of elastomeric material, the unitary sheet formed to at least partially encircle and generally conform to a joint of a wearer, wherein the layer includes an inner surface having one or more channels defined therein, each channel including one or more undercuts.

The terms "comprises" and variations thereof do not have a limiting meaning where these terms appear in the description and claims. Such terms will be understood to imply the inclusion of a stated step or element or group of steps or elements but not the exclusion of any other step or element or group of steps or elements. By "consisting of' is meant including, and limited to, whatever follows the phrase "consisting of." Thus, the phrase "consisting of' indicates that the listed elements are required or mandatory, and that no other elements may be present. By "consisting essentially of' is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that other elements are optional and may or may not be present depending upon whether or not they materially affect the activity or action of the listed elements.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

In this application, terms such as "a", "an", and "the" are not intended to refer to only a singular entity, but include the general class of which a specific example may be used for illustration. The terms "a", "an", and "the" are used interchangeably with the term "at least one." The phrases "at least one of' and "comprises at least one of' followed by a list refers to any one of the items in the list and any combination of two or more items in the list.

As used herein, the term "or" is generally employed in its usual sense including "and/or" unless the content clearly dictates otherwise.

The term "and/or" means one or all of the listed elements or a combination of any two or more of the listed elements.

Also herein, all numbers are assumed to be modified by the term "about". As used herein in connection with a measured quantity, the term "about" refers to that variation in the measured quantity as would be expected by the skilled artisan making the measurement and exercising a level of care commensurate with the objective of the measurement and the precision of the measuring equipment used.

Also herein, the recitations of numerical ranges by endpoints include all numbers subsumed within that range as well as the endpoints (e.g., 1 to 5 includes 1, 1.5, 2, 2.75, 3, 3.80, 4, 5, etc.).

As used herein as a modifier to a property or attribute, the term "generally", unless otherwise specifically defined, means that the property or attribute would be readily recognizable by a person of ordinary skill but without requiring absolute precision or a perfect match (e.g., within +/- 20 % for quantifiable properties). The term "substantially", unless otherwise specifically defined, means to a high degree of approximation (e.g., within +/- 10% for quantifiable properties) but again without requiring absolute precision or a perfect match. Terms such as same, equal, uniform, constant, strictly, and the like, are understood to be within the usual tolerances or measuring error applicable to the particular circumstance rather than requiring absolute precision or a perfect match.

The above summary of the present disclosure is not intended to describe each disclosed embodiment or every implementation of the present disclosure. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### BRIEF DESCRIPTION OF THE DRAWINGS

FIG. 1 is an isometric view of an orthopedic support according to one embodiment of the present disclosure, highlighting the posterior area of the support;
FIG. 2 is an isometric view of the orthopedic support of FIG. 1, highlighting the anterior area of the support;
FIG. 3 is a posterior view of the orthopedic support of FIG. 1-2;
FIG. 4 is a side view of the orthopedic support of FIG. 1;
FIG. 5 is an end view of the orthopedic support of FIG. 1;
FIG. 6 is an isometric view of an orthopedic support according to another implementation of the present disclosure; and
FIG. 7 is an isometric view of an orthopedic support according to the present invention.

While the above-identified figures set forth several embodiments of the disclosure other embodiments are also contemplated, as noted in the description. It should be understood that numerous other modifications and embodiments can be devised by those skilled in the art, which fall within the scope invention which is defined in the appended claims.

### DETAILED DESCRIPTION OF ILLUSTRATIVE EMBODIMENTS

An orthopedic support of the present disclosure is generally configured to conform to a body part or joint for support and compression purposes. In general, the present orthopedic supports are suitable for embracing and supporting a body part of a human or animal, typically comprising a joint or articulation. Examples of said body parts are limbs such as an arm, a leg, a foot, a hand, etc., the joints or articulations of these limbs being an elbow, a knee, an ankle, a hip, a wrist, etc. Other possible body parts comprising an articulation may be neck, shoulder, back, collar, etc. In presently preferred embodiments the supports are configured as wrist supports. The orthopedic supports may also comprise other components, such as at least one adjustable closure system for adjusting the fit and level of support provided, as well as semi-rigid (or rigid) splint elements. As used in this Specification, the term "supports" is intended to have a broad application and includes any device for interacting with a joint, muscle or bone of the human body for therapeutic purposes, including supports and braces, as should be appreciated by one skilled in the art.

The orthopedic supports of the present disclosure comprise a single, unitary sheet of elastomeric material. The sheet of material may be shaped into a configuration approximating the contour of at least a portion of a potential wearer's anatomy. Such contoured configuration can be well suited for being applied to e.g. a wrist, a wrist with thumb, hip, knee, shoulder, back, neck, collar (e.g. in the form of a corset), and head (in the form of a helmet) etc. In presently preferred implementations, the sheet of material is shaped into the desired contour by the manufacturer (i.e., the wearer is not required to set the shape of the material). In presently preferred circumstances, the orthopedic supports lacks fabric, cotton, or any other textile material either on the exterior surface of the support or between the wearer's skin and the support. Due to the lack of any textile or other absorbent layer of material, the orthopedic supports are at least washable and/or submergible, and can be essentially waterproof.

The elastomeric material used in the supports of the present disclosure is preferably a thermoformable plastic material. Suitable thermoformable plastic materials include polyurethanes (especially polyurethanes based on semi-crystalline polyester polyols), polyethylene, ethylene vinyl acetate (EVA), cis and trans polyisoprene, polyesters such as polycaprolactone and the like, and combinations thereof. The presently preferred thermoformable material for use in the present disclosure is preferably a foam, such as a urethane foam, foam rubber, or EVA foam. EVA is durable plastic foam material made of ethylene vinyl acetate resin. EVA based materials are sufficiently deformable and flexible, but still offer adequate rigidity for an orthopedic support. EVA typically feels soft on the skin, does not induce allergic reactions on the skin and is X-ray translucent. In alternative embodiments, the elastomeric material is a thermoformable silicone.

In certain embodiments, the elastomeric material in the formed orthopedic support has an Asker C hardness between 20 and 60, in other embodiments between 25 and 50, and in yet other embodiments between 30 and 45, and for instance of 35. An Asker C hardness is a measure of the resistance of the material toward indentation by an indenter, and is typically used to characterize the relative hardness of relatively soft elastomers such as rubber or soft plastics. Asker C hardness can be measured by a conventional durometer.

The elastomeric materials of the orthopedic supports of the present disclosure are relatively flexible and conformable, but are relatively resistant to elongation once formed into an orthopedic support. Such substantial stretch resistance can improve the strength and durability of the orthopedic support, while simultaneously enabling the use of fenestrations, recesses, and channels to be created in the body of the support without introducing fracture lines or undesired wear in the material. As used herein, a relatively stretch resistant material has a percent elongation at break of no greater than 350 percent, though presently preferred materials have a percent elongation at break of no greater than 300 percent.

Suitable elastomeric materials in the supports of the present disclosure can also feature high tear resistance, as exemplified by a tear strength of at least 1.0 N/mm², measured as the maximum tension the material can withstand without tearing. In other embodiments, the tear strength of the material is at least 1.5 N/mm², in other embodiments at least 2.0 N/mm², and it yet other embodiments at least 2.5 N/mm². Suitable elastomeric materials can also exhibit a percent elongation (i.e., elasticity) of at least 20, in other embodiments at least 30, and in other embodiments at least 35. In certain desirable implementations, the stretchable material exhibits a percent elongation of less than 65, in other embodiments less than 60, and in yet other embodiments less than 50.

In typical embodiments, the orthopedic supports of the present disclosure have an Asker C hardness which is substantially similar over the complete surface of the support; the supports accordingly demonstrate a homogenous hardness. In presently preferred circumstances, the support comprise a single, unitary sheet of elastomeric material that has substantially homogenous material properties throughout its thickness. A substantially homogeneous sheet will consist of a single strata of elastomeric material and will lack individually modified or engineered layers having different physical or chemical properties within the thickness of that strata. The unitary, substantially homogeneous sheet may still incorporate thick and thin areas in different portions, or may include recesses, perforations and larger apertures.

Orthopedic supports of the present disclosure can be advantageously washable and/or waterproof. Such supports lack textile or other absorbent material (e.g., cotton fabric and other natural/synthetic textiles) between the skin and the support that retain moisture, which can be uncomfortable or cause maceration after absorbing moisture, such that the inner surface of the elastomeric material is in direct contact with the wearer's skin. The supports will typically not deteriorate or swell when wet. Such supports can air dry in a brief time due to their non-absorbent properties and can be more quickly dried with a hand dryer or towel. This is a particular advantage in terms of hygiene, occupational utility, recreational use, sporting use, and longer term wear. The orthopedic supports can accordingly be worn and used in a variety of activities without outsized fear of support damage or wearer discomfort.

Orthopedic supports may also incorporate at least one of a number of actives including for example, medicaments, anti-infective agents, antimicrobials, antiseptics, and fragrances.

The orthopedic supports according to the present disclosure can also include one or more ventilation apertures. These apertures permit and facilitate ventilation of the skin when the brace is placed upon and in contact with a body part. Preferably, between about 35-85% of the total surface area of the support includes ventilation apertures. This amount of perforation will typically provide adequate strength for the support body while allowing for good air circulation, which will improve skin ventilation as compared to non-perforated and/or non-breathable supports. Alternatively, apertures over a smaller percentage of surface area will provide only slight ventilation if desired. The ventilation apertures are generally uniform and have a largest cross-sectional dimension generally in the range of about 1.02 mm to 15.2 mm (0.04 inches to 0.6 inches). In certain circumstances, it may be desirable to omit ventilation apertures proximate edge regions of the support.

The relative volume defined by the apertures can vary depending on the design and desired treatment from the orthopedic support. In certain implementations, the elastomeric material comprises at least 50%, at least 60%, or at least 70% of the total volume of the orthopedic support, with the remaining portion of the volume residing in the apertures. For implementations requiring greater support and/or coverage of the wearer's anatomy in addition to apertures, the elastomeric material can comprise at least 90%, at least 95%, or at least 98% of the total volume of the support. In other alternative implementations, the elastomeric material comprises no greater than 40%, no greater than 25%, no greater than 15% of the total volume of the support, with the remaining portion residing in the apertures. The volume of the support can be established, for example, by comparing the volume of water displaced by the ventilated support to the volume displaced by a support shell lacking apertures.

Similarly, the comparative surface area between apertures and elastomeric material can vary depending on the desired support design and treatment aims. In certain implementations, the elastomeric material comprises at least 40%, at least 50%, or at least 60%, at least 70%, at least 80% of the total surface area of the orthopedic support, with the remaining portion of the surface area residing in the apertures. For implementations requiring greater support and/or coverage of the wearer's anatomy in addition to apertures, the elastomeric material can comprise at least 90%, at least 95%, or at least 98% of the total surface area of the support. In other alternative implementations, the elastomeric material comprises no greater than 40%, no greater than 25%, no greater than 15% of the total surface area of the support, with the remaining portion residing in the apertures.

A particularly suitable orthopedic support featuring a preformed, substantially homogeneous elastomeric material is a wrist brace. Wrist braces of the present disclosure typically include a main brace body with a semi-rigid splint disposed at least partially therein. The main brace body generally includes a unitary sheet of elastomeric material that is contoured for wrapping about portions of a wearer's hand and forearm to enclose the wearer's wrist. Such a main brace body could be applied to a wearer's hand and wrist in a fashion similar to pulling on a glove. In other implementations, portions of the body may self-move away from one another, allowing a user to press his or her hand and forearm into the brace body. Typically, the main brace body is formed from a single piece of material that wraps around the wearer's wrist and hand, forming two ends with opposing sides that are drawn towards each other (or possibly even overlap) on the posterior of the wearer's wrist and hand when the wrist brace is applied.

A semi-rigid splint can be disposed at least partially within or on the main brace body. The splint is designed to hold the wrist in neutral position when the wrist brace is worn and is typically more rigid than the main brace body. The splint can comprise any material that is rigid enough to provide support to the hand and wrist. The splint typically comprises a semi-rigid plastic or metal, though other materials may be used, depending on the desired stiffness or flexibility of the support. The splint can be attached to or at least partially imbedded within the main brace body using methods known in the art. In some embodiments, the splint is removably coupled to an outer surface of the brace through use of mating components. In other particular implementations, the splint is defined by a relatively thicker section of the main brace body and is not otherwise detachable from the support. Other stiffening elements (stays, splints, etc.) may be coupled to brace body as desired.

The unitary sheet of elastomeric material defining the wrist brace is typically, relatively thin to enhance conformance with the wearer's wrist and forearm. For example, it may be preferred that the brace be formed of elastomeric materials with a maximum thickness of 9.5 mm (0.375 inches), in some embodiments 4.763 mm (0.1875 inches) or less, and in other embodiments no greater than 3.175 mm (0.125 inches). It may be desirable in implementations featuring channels or other indentations in the surface of the brace to select a relative thicker maximum thickness to ensure brace integrity.

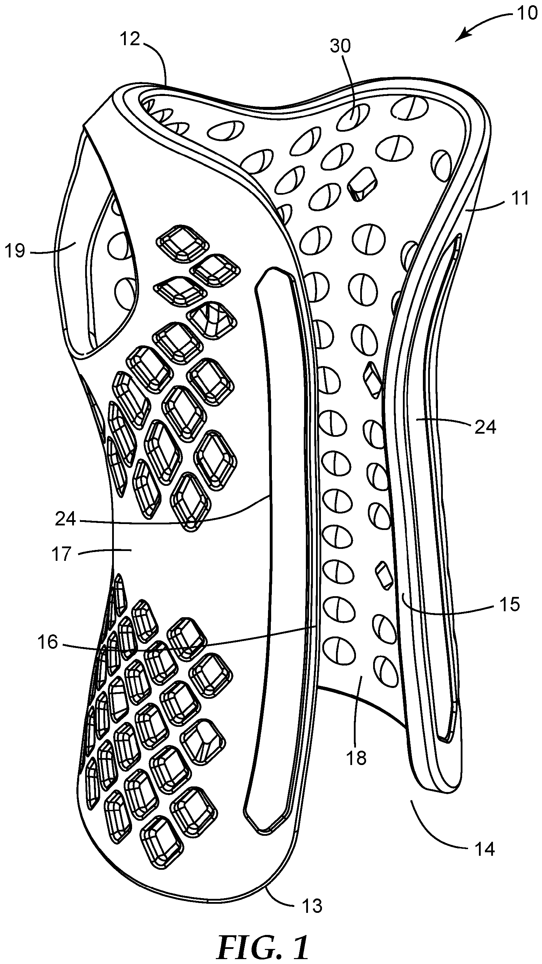

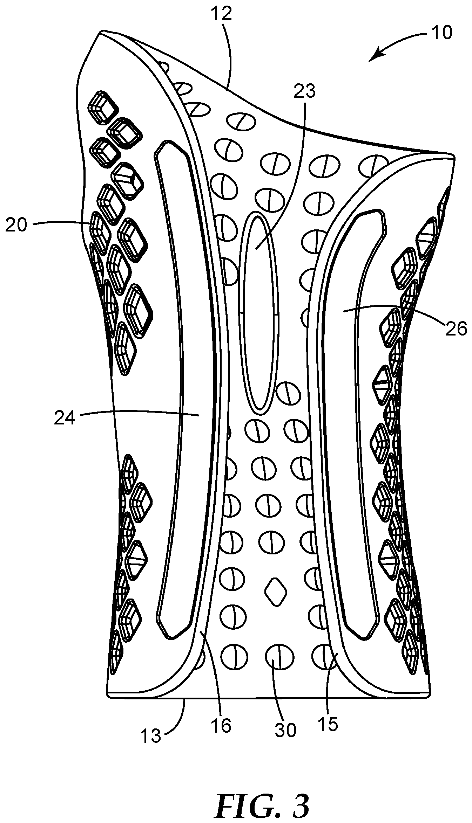

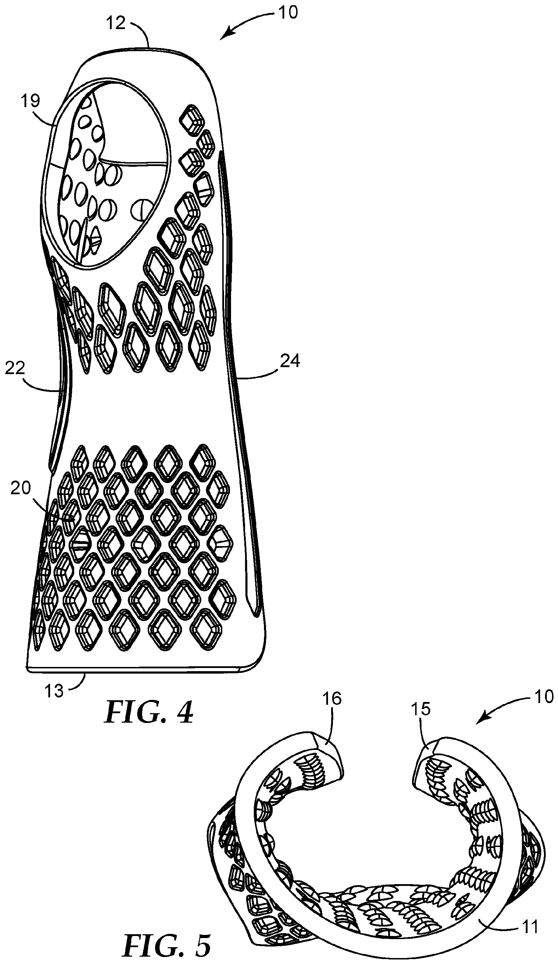

An exemplary preformed wrist brace 10 including the elastomeric materials of the present disclosure is illustrated in FIGS. 1-5. The wrist brace 10 includes a main brace body 11 that is preformed into the general contour of a wearer's wrist and exhibits a generally semi-cylindrical shape. The main brace body 11 includes a proximal edge 12, a distal edge 13, and a pair of opposing lateral edges 15 and 16 extending there between. The lateral edges 15, 16 are separated by a space that can increase or decrease depending on the size of the wearer's arm, allowing the wearer to don and to remove the brace 10 with relative ease. The distal edge 13 is substantially linear (though contoured), while the proximal edge 12 includes an additional curvature that arcs proximate to a thumb hole 19, as shown in Figure 3. The contour of the distal edge 13 provides a distal opening 14. Though not depicted herein, a main brace body 12 can be provided without a thumb hole, such that a portion of the proximal edge rests below the wearer's thumb.

The main body 11 includes an exterior surface 17 and interior, skin facing surface 18, each comprised of an elastomeric material. In presently preferred circumstances, the main brace body 11 consists of a single, unitary sheet of elastomeric material having substantially homogeneous properties throughout the thickness of the body 11. Advantageously, neither the inner surface nor the outer surface includes natural or synthetic textile material, or blends thereof: the inner surface 18 is directly in contact with the skin.

The inner surface 18 can be provided with a plurality of protrusions 30 in the area of the body 11. The protrusions 30 project outwardly from the inner surface 18 and accordingly displace portions of the main body 11 away from the wearer's skin. For some wearers, the reduction in surface area in contact with the skin offered by the protrusions may be desirable. Though depicted as domes, other protrusion shapes may be suitable for reducing the contact area of the inner surface can include, but not limited to, a variety of polyhedral shapes, parallelepipeds, prismatoids, prismoids, etc., and combinations thereof. For example, the features can be polyhedral, conical, frusto-conical, pyramidal, frusto-pyramidal, spherical, partially spherical, hemispherical, ellipsoidal, dome-shaped, cylindrical, and combinations thereof. In one alternative, the protrusions comprises a series of rails extending between the proximal and distal edges 12, 13. It may be preferred that the anatomy contacting surfaces of the protrusions 30 are rounded or softened for comfort under compression.

The exterior surface 17 features a plurality of ventilation apertures 20. In the depicted embodiment, the apertures 20 are generally diamond shaped, however any other shape may be used as desired. The ventilation apertures 20 extend through the thickness of the main brace body, fluidly coupling the exterior and interior surfaces 17, 18. The apertures 20 permit and facilitate ventilation of the skin when the brace is placed upon and in contact with the wearer's wrist and forearm. In braces consistent with the depicted embodiment, the apertures comprise at least 20% and no greater than 35% of the total brace volume. In other potentially advantageous wrist braces, the apertures comprise no greater than 5%, 4%, 3%, 2%, or 1% of the total brace volume, such that the majority of the brace is defined by the elastomeric material.

The exterior surface 17 further includes recessed areas 24 directly adjacent each side edge 15, 16. The recessed areas 24 are sized and shaped to accept components of an adjustable closure system. The adjustable closure system (not shown) allows the wearer to modify the fit of the brace 10 by controlling the relative distance between side edges 15 and 16 of the main brace body 11. This closure system may be a mechanical closure system consisting of, but not limited to, hook and loop fasteners, adjustable straps, snaps, laces, toothed zip ties, ratchet lace systems, ski boot type buckles, combinations thereof, and the like. These preferably reversible adjustment systems allow the wearer to loosen and tighten the brace 10 if there is discomfort or insufficient support.

One adjustable closure system in particular includes a ratchet and cable system. The system can utilize a series of guide members on opposite sides of the main brace body 11. A lace is laced through guide members on alternate sides of the brace body 11. The lace can be drawn tight so the main brace body 11 has the appropriate tension on wearer's wrist and forearm. A reel mechanism can be used to mechanically tighten and lock the tightened lace that can be easily released and tightened with micro adjustments. Examples of various ratchet and lace tightening systems suitable for this task are disclosed in greater detail in U.S. Patent Publication No 2012/0004587 (Nickel et al.).

The adjustable closure system component, such as the plurality of lace or cable guides integrally arranged on a unitary housing piece as described in US Publication No. 2012/0004587 (Nickel et al.), may be secured in recess 24 by any means known in the art. In some implementations, the component is releasably secured, through interference fit with recessed area 24 or through other mating elements (e.g., cooperating post and aperture as described in US Patent No. 7,867,182 (Iglesias et al.)). In other implementations, the component may be permanently secured via adhesive, welding, ultrasonic bonding, or like attachment varieties known in the art.

By attaching them to the main body separately, the components of the adjustable closure system may be made from a different material than the main brace body. For example, the adjustable closures system components may be formed of a harder, more durable material than the main brace body 11. In such instances, the component may provide additional support to the wearer's wrist.

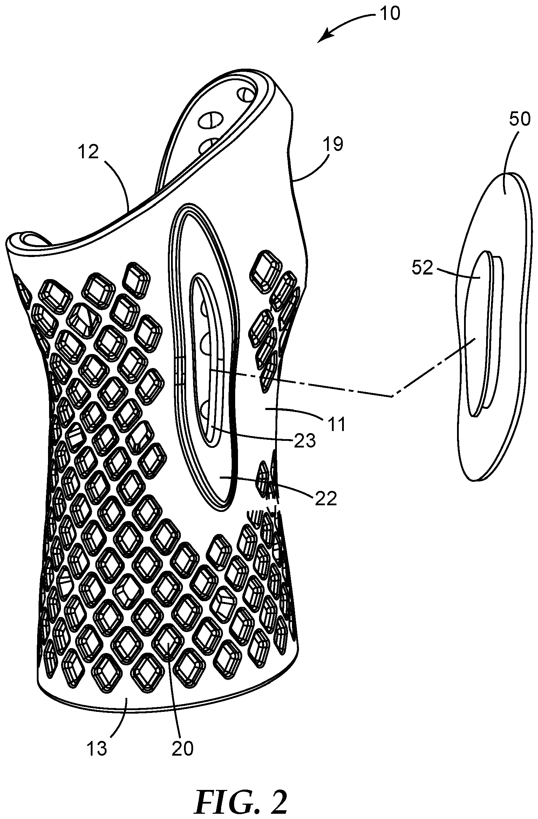

Turning to FIG. 2, the anterior area of the main brace body 11 can include a semi-rigid splint 50. The splint 50 material may also be chosen to provide some flexibility to permit limited flexion and extension movement of the wearer's wrist. The splint 50 is shaped with a curvature to conform to a portion of the palm and wrist of the wearer to maintain the hand slightly in extension relative to the wrist. The splint may be removably attached to the body 11 via recess 22 and aperture 23. The splint 50 can include a projection 52 that may be inserted into compatible apertures 23 to secure the splint 50 to the body. In other alternatives, the splint may include one or more apertures, and the exterior 17 can be provided with cooperating posts or other projections. The presently preferred splint comprises molded plastic, although the splint could be made from a wide variety of materials, including metals for extra stiffness.

In alternative implementations, the main body 12 may incorporate thick and thin areas in different portions of the brace, in order to provide additional support in some areas and greater flexibility in other areas. In particular, the splint 50 may be provided as a relatively thicker area of the body 11 material on the anterior of the wrist. Furthermore, the splint may be created by embedding molded plastic or metal in the main body.

In one method of donning the wrist brace 10, the lateral edges 15, 16 may be displaced by a degree sufficient for the wearer to lower his or her wrist into the brace in a direction towards the anterior inner surface. In other aspects, lateral edges 15, 16 may open sufficiently for a wearer to insert his or her hand and wrist into distal opening 14 and through to proximal end 15, with the wearer's thumb being inserted into optional thumb hole 19.

As previously suggested, the orthopedic supports disclosed herein can be made of a thermoplastic or thermoset foam material. Manufacturing supports using such a foam material can include providing a resin that includes a pre-mixture of resin, pigment, and a growth additive (e.g., ENGAGE polyolefins, available from Dow Chemical, Midland MI), among other processing additives. The resin, originally in pellet form, is heated to a liquid state. This liquid resin is introduced into a mold that has been heated prior to receiving the resin. The volume of resin injected into the mold is controlled in certain implementations by the pitch of the screw that drives the liquid resin into the mold. The liquid resin is allowed to set, at which time the mold is opened and the formed support is removed from the mold. The formed support is then placed on a cooling last, where it is allowed to air dry.

During this process, a relatively small support confirming to the size of the mold is created, but when the mold opens, the support springs out as it expands in size. Then, as the support is air cooled, it contracts to a final size. Thus, the process involves use of both an expansion characteristic and a contraction characteristic. Multiplying the size of the support in the mold by the expansion characteristic yields the size of the support after the mold is opened. Multiplying the expansion characteristic by the contraction characteristic provides a final growth value representative of the final size of the support relative to the mold.

In such a manufacturing process, a number of elements can be controlled to achieve the desired end result. These elements include but are not limited to, the volume of material introduced into the mold, the size of the mold, the composition of the material being used, the temperature of the mold, the cure time, and the size of the cooling structure. Thus, for example, to create two different sized supports from the same mold, one volume of a material is screwed into a mold to create one support size, and another volume of the same material is screwed into the same mold to create a different support size. Once removed from the mold, the supports are cooled on cooling structures of different sizes. Thus, the process uses a modified volume and cooling structure size to control the end product, while keeping the mold size and the composition fixed.

While the above approach creates supports of different sizes, control of the final sizes can, in certain circumstances, be somewhat limited and/or unpredictable. In part to address this, one skilled in the art may instead use a fixed volume and composition of material, and cooling structure size, while varying mold sizes to control the size of the end product. This approach is particularly valuable for supports manufactured of the same color resin. Where different colors are involved, the composition of the resin may be varied across the colors to achieve size control between colors. This composition adjustment is more fully described below.

In particular embodiments of the present disclosure, the resin is an EVA copolymer base material. Additives are included with the EVA base to create an expansible and cross-linking material. More particularly, an expanding powder is added which decomposes at a specific temperature to produce gases which cause the material to rise as it sets within a mold. Accordingly, when the mold is opened, an instantaneous expansion of the molded part results. During this expansion, the dimensions of the part increase rapidly, while the proportions and shape remain reasonably constant providing a consistent shape of the end part relative to the original mold. Additional disclosure of such cross-linking and expansion processes are provided in European Patent 0 802 039 A2 and US Patent No. 6,993,858, including certain viable cross-linking agents and growth additives.

Certain presently preferred embodiments of the present disclosure utilize EVA and polyolefin closed cell foams available commercially under the POLYCELL brand from MDI Products, Sebastian, FL. This material can provide a relatively soft support piece that has appropriate size reproducibility and durability.

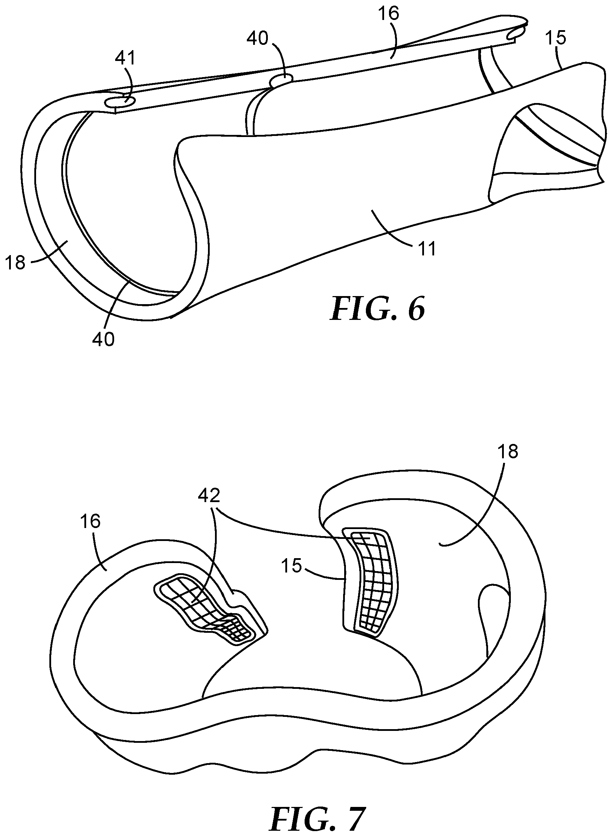

Advantageously, use of a process resulting in growth upon release of the mold enables the creation of recesses, depressions, and channels of various geometries in an orthopedic support. In one particularly advantageous implementation, the support body is provided with one or more undercuts. An exemplary illustration of a particularly advantageous undercut is depicted in FIG.6. The interior surface 18 of a main brace body 11 includes a plurality of laterally extending channels 40. The channels 40 include a generally T-shaped cross section, resulting in regions 41 of the channel 40 that reside between the exterior and inner surfaces 17, 18. As depicted, the channels 40 present an entrance at lateral edge 15 and extend at least partially across the surface of the body towards the opposite lateral edge 16. The opposing lateral edge 16 may also include an entrance to the channel 40, if desired. The channels 40 can useful for, in some exemplary implementations, receiving components of the adjustable closure system. For example, the wearer may thread a portion of a lace or strap into the channel 40 once the brace is donned, obscuring extraneous system components from view and providing a sleeker exterior appearance.

Another exemplary use of undercuts is illustrated in FIG. 7. Recessed areas 42 are disposed proximate each lateral edge 15, 16 of the brace body 11. Like channel 40, the recessed areas include undercut regions that reside between the exterior and inner surfaces 17, 18 of the main brace body 11. Recessed areas 42, unlike channels 40, extend along the longitudinal axis of the brace 10, between the proximal and distal edges 12, 13. The recessed areas 42 may be used similarly to recesses 24 on the exterior surface by, e.g., attaching additional stiffening elements to provide rigid support to the brace body or to removably house components of an adjustable closure system. In one exemplary embodiment, a unitary housing piece featuring one or more lace guides may be placed in either recessed area 42. In another exemplary embodiment, a recessed area on the inner, anterior side of the brace may be used to attach a splint 50. Channels, recessed areas, and undercuts may also be used to create regions of differing support in the brace body by thickness and/or rigidity.

While orthopedic supports can be molded as previously described, based on the disclosure provided herein, one of ordinary skill in the art will appreciate that various embodiments of the present disclosure can be utilized in relation to other molding processes, and or assembly methods. For example, a plastic orthopedic support could be injection molded using techniques known in the art. As another example, the support could be Freon cooled, rather than air cooled in order to speed the manufacturing process. As yet another example, a stay or other stiffening element may be co-molded with the support body.

Various modifications and alterations to this invention will become apparent to those skilled in the art without departing from the scope of this invention defined by the claims. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. An orthopedic support comprising:
a flexible main brace body (11) comprising first and second opposing sides, a proximal edge (12), a distal edge (13), and two lateral edges (15, 16), the brace body consisting essentially of a unitary sheet of elastomeric thermoset material, the unitary sheet thermoformed to at least partially encircle and generally conform to a joint of a wearer,
wherein the brace body (11) includes an inner surface (18) and an exterior surface (17),
wherein the brace body (11) includes a plurality of engineered apertures (20, 23) extending through the sheet from the exterior surface (17) to the inner surface (18), and
wherein the unitary sheet comprises a recessed area (42) disposed proximate to each of the lateral edges (15, 16), the recessed areas (42) disposed on an inner surface (18) of the body extending along the longitudinal axis of the brace body (11) between the proximal and distal edges (12, 13), wherein the recessed areas (42) further comprise undercut regions.

2. The support of claim 1, further comprising:
one of (a) an adjustable closure system or (b) a stiffening element disposed in the recessed area.

3. The support of claim 1, wherein the thermoset material is a closed cell foam.

4. The support of claim 3, wherein the foam is crosslinked.

5. The support of claims 1-4, wherein the thermoset material exhibits an Asker C hardness of at least 20 and no greater than 60.

6. The support of claim 5, wherein the thermoset material exhibits an Asker C hardness of at least 30 and no greater than 55.

7. The support of any of the previous claims, wherein the inner surface is not coupled to a textile material, and when donned is in contact with an outer surface of the wearer.

8. The support of any of the previous claims, and further comprising a structural support removably received in the exterior surface of the main body, the structural support having a stiffness greater than the stiffness of the unitary sheet.

9. The support of any of the previous claims, wherein the main brace body includes a fastener adapted to draw the opposing sides towards one another, thereby providing tension on the main brace body.

## Patentansprüche

1. Orthopädische Stütze, die aufweist:
einen flexiblen Hauptstützkörper (11), der erste und zweite gegenüberliegende Seiten, eine proximale Kante (12), eine distale Kante (13) und zwei seitliche Kanten (15, 16) aufweist, wobei der Stützkörper im Wesentlichen aus einem einstückigen Bahnenmaterial aus elastomerem Duroplastmaterial besteht, wobei das einstückige Bahnenmaterial thermogeformt ist, um ein Gelenk eines Trägers mindestens teilweise zu umgeben und sich allgemein daran anzupassen,
wobei der Stützkörper (11) eine innere Oberfläche (18) und eine äußere Oberfläche (17) aufweist,
wobei der Stützkörper (11) eine Mehrzahl von konstruierten Öffnungen (20, 23) aufweist, die sich von der äußeren Oberfläche (17) zu der inneren Oberfläche (18) durch das Bahnenmaterial erstrecken, und
wobei das einstückige Bahnenmaterial einen nahe jeder der seitlichen Kanten (15, 16) angeordneten vertieften Bereich (42) aufweist, wobei die auf einer inneren Oberfläche (18) des Körpers angeordneten vertieften Bereiche (42) sich entlang der Längsachse des Stützkörpers (11) zwischen den proximalen und distalen Kanten (12, 13) erstrecken, wobei die vertieften Bereiche (42) ferner unterschnittene Regionen aufweisen.

2. Stütze nach Anspruch 1, ferner aufweisend:
eines von (a) einem verstellbaren Verschlusssystem oder (b) einem Versteifungselement, das in dem vertieften Bereich angeordnet ist.

3. Stütze nach Anspruch 1, wobei das Duroplastmaterial ein geschlossenzelliger Schaumstoff ist.

4. Stütze nach Anspruch 3, wobei der Schaumstoff vernetzt ist.

5. Stütze nach den Ansprüchen 1-4, wobei das Duroplastmaterial eine Asker-C-Härte von mindestens 20 und nicht größer als 60 hat.

6. Stütze nach Anspruch 5, wobei das Duroplastmaterial eine Asker-C-Härte von mindestens 30 und nicht größer als 55 hat.

7. Stütze nach einem der vorstehenden Ansprüche, wobei die innere Oberfläche nicht an ein Textilmaterial gekoppelt ist und, wenn sie angelegt wird, in Kontakt mit einer äußeren Oberfläche des Trägers ist.

8. Stütze nach einem der vorstehenden Ansprüche, und ferner aufweisend eine abnehmbar in der äußeren Oberfläche des Hauptkörpers aufgenommene strukturelle Stütze, wobei die strukturelle Stütze eine Steifigkeit größer als die Steifigkeit des einstückigen Bahnenmaterials aufweist.

9. Stütze nach einem der vorstehenden Ansprüche, wobei der Hauptstützkörper ein Befestigungsmittel aufweist, das angepasst ist, um die gegenüberliegenden Seiten aufeinander zu zu ziehen, wodurch Spannung an dem Hauptstützkörper bereitgestellt wird.

## Revendications

1. Support orthopédique comprenant :
un corps d'attelle principal souple (11) comprenant des premier et deuxième côtés opposés, un bord proximal (12), un bord distal (13), et deux bords latéraux (15, 16), le corps d'attelle constitué sensiblement d'une feuille unitaire de matériau élastomère thermodurcissable, la feuille unitaire thermoformée pour encercler au moins partiellement et se conformer globalement à une articulation d'un porteur,
dans lequel le corps d'attelle (11) inclut une surface interne (18) et une surface externe (17),
dans lequel le corps d'attelle (11) inclut une pluralité d'ouvertures modifiées (20, 23) s'étendant à travers la feuille depuis la surface extérieure (17) vers la surface interne (18), et
dans lequel la feuille unitaire comprend une zone évidée (42) disposée à proximité de chacun des bords latéraux (15, 16), les zones évidées (42) disposées sur une surface interne (18) du corps s'étendant le long de l'axe longitudinal du corps d'attelle (11) entre les bords proximal et distal (12, 13), dans lequel les zones évidées (42) comprennent en outre des régions en contre-dépouille.

2. Support selon la revendication 1, comprenant en outre :
l'un (a) d'un système de fermeture réglable ou (b) d'un élément de raidissement disposé dans la zone évidée.

3. Support selon la revendication 1, dans lequel le matériau thermodurcissable est une mousse à cellules fermées.

4. Support selon la revendication 3, dans lequel la mousse est réticulée.

5. Support selon les revendications 1 à 4, dans lequel le matériau thermodurcissable présente une dureté Asker C d'au moins 20 et non supérieure à 60.

6. Support selon la revendication 5, dans lequel le matériau thermodurcissable présente une dureté Asker C d'au moins 30 et non supérieure à 55.

7. Support selon l'une quelconque des revendications précédentes, dans lequel la surface interne n'est pas couplée à un matériau textile, et lorsqu'elle est revêtue est en contact avec une surface externe du porteur.

8. Support selon l'une quelconque des revendications précédentes, et comprenant en outre un support structurel reçu de manière amovible dans la surface extérieure du corps principal, le support structurel ayant une rigidité supérieure à la rigidité de la feuille unitaire.

9. Support selon l'une quelconque des revendications précédentes, dans lequel le corps d'attelle principal inclut une fixation adaptée pour tirer les côtés opposés l'un vers l'autre, fournissant de ce fait une tension sur le corps d'attelle principal.
